(19) Europäisches Patentamt / European Patent Office / Office européen des brevets

(11) **EP 4 613 129 A1**

(12) **EUROPEAN PATENT APPLICATION**
published in accordance with Art. 153(4) EPC

(43) Date of publication:
**10.09.2025 Bulletin 2025/37**

(21) Application number: **22964406.7**

(22) Date of filing: **02.11.2022**

(51) International Patent Classification (IPC):
***A24F 40/46*** *(2020.01)*

(52) Cooperative Patent Classification (CPC):
**A24F 40/46**

(86) International application number:
**PCT/JP2022/040964**

(87) International publication number:
**WO 2024/095386 (10.05.2024 Gazette 2024/19)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Japan Tobacco Inc.
Tokyo 105-6927 (JP)**

(72) Inventor: **SAKAI, Ryuji
Tokyo 130-8603 (JP)**

(74) Representative: **Hoffmann Eitle
Patent- und Rechtsanwälte PartmbB
Arabellastraße 30
81925 München (DE)**

(54) **AEROSOL GENERATION DEVICE**

(57)     [Problem] To provide an aerosol generation device having further improved waterproofing properties.

[Solution] An aerosol generation device comprising: a holding part having a cylindrical structure, an aerosol-generating substrate being inserted into the interior of the cylindrical structure; a heat-emitting part that protrudes into the cylindrical structure through a bottom surface of the cylindrical structure from the exterior thereof, the heat-emitting part heating the aerosol-generating substrate; a flange part that juts out from the heat-emitting part at the exterior of the cylindrical structure in a direction orthogonal to an extension direction of the cylindrical structure; a securing part that grips the flange part in the extension direction of the heat-emitting part; and a hermetic member that is provided between the flange part and the securing part, the hermetic member hermetically sealing a gap communicating from the interior of the cylindrical structure along the heat-emitting part.

FIG. 2

## Description

[TECHNICAL FIELD]

The present invention relates to an aerosol generation device.

[BACKGROUND ART]

Inhalation devices such as e-cigarettes and nebulizers that generate substances to be inhaled by a user are in widespread use. Inhalation devices are capable of generating an aerosol by heating an aerosol source. This allows the user to inhale the aerosol generated by the inhalation device to enjoy the flavor of the aerosol.

For example, the inhalation device can generate an aerosol by inserting a blade-shaped or pin-shaped heater into a stick-type substrate comprising an aerosol source and heating the stick-type substrate from the inside. In an inhalation device comprising such a heater, the heater protrudes inside the cup through the cup receiving the stick-type substrate, creating a gap around the protruding heater.

It is disclosed in the following PTL 1 that a bushing is provided between the flange side surface of the blade-type heater and the housing to ensure that liquid produced by condensation of the aerosol does not leak into the interior of the inhalation device through a gap around the blade-type heater.

[CITATION LIST]

[PATENT LITERATURE]

[PTL 1] JP 2020-528737 A

[SUMMARY OF INVENTION]

[TECHNICAL PROBLEM]

However, in the above-mentioned PTL 1, there was room for further enhancement of the waterproofing properties of the inhalation device, as no detailed examination has been made on the position of waterproof members such as bushings. Furthermore, if the inside of the cup receiving the stick-type substrate is washed with water, a large amount of the water will be poured into the cup. Therefore, there was a need to improve waterproofing properties in the gap around the protruding heater.

Accordingly, the present invention has been made in view of the above problems, and the object of the present invention is to provide a novel and improved aerosol generation device that can have further improved waterproofing properties.

[SOLUTION TO PROBLEM]

To solve the above problem, one aspect of the present invention provides an aerosol generation device comprising: a holding part having a cylindrical structure, an aerosol-generating substrate being inserted into the interior of the cylindrical structure; a heat-emitting part that protrudes into the cylindrical structure through a bottom surface of the cylindrical structure from the exterior thereof, the heat-emitting part heating the aerosol-generating substrate; a flange part that juts out from the heat-emitting part at the exterior of the cylindrical structure in a direction orthogonal to an extension direction of the cylindrical structure; a securing part that grips the flange part in the extension direction of the heat-emitting part; and a hermetic member that is provided between the flange part and the securing part, the hermetic member hermetically sealing a gap communicating from the interior of the cylindrical structure along the heat-emitting part.

The hermetic member may generate compressive stress in the extension direction of the heat-emitting part.

The hermetic member may be made of an elastic material or a metallic material.

The hermetic member may be ring-shaped.

The diameter of the hermetic member may be 1-8 mm.

A width between the flange part of the gap and the securing part may be less than or equal to a thickness of the hermetic member.

The thickness of the hermetic member may be 1 mm or less.

The securing part may include a first securing part spaced apart from the heat-emitting part and provided on the same side as the holding part with respect to the flange part, and a second securing part provided on the opposite side of the holding part with respect to the flange part and gripping the heat-emitting part in a direction orthogonal to the extension direction of the heat-emitting part.

The flange part may be between the first securing part and the second securing part.

[ADVANTAGEOUS EFFECTS OF INVENTION]

According to the present invention, as described above, it is possible to further increase the waterproofing properties of the aerosol generation device.

[BRIEF DESCRIPTION OF DRAWINGS]

[Fig. 1] Fig. 1 is a schematic diagram illustrating schematically a configuration example of an inhalation device.
[Fig. 2] Fig. 2 is a schematic cross-sectional view showing a first configuration example of a support structure of a heat-emitting part.

[Fig. 3] Fig. 3 is a schematic cross-sectional view showing a second configuration example of the support structure of the heat-emitting part.

[Fig. 4] Fig. 4 is a schematic diagram showing a model for deriving the tilt of the heat-emitting part when a hermetic member is not provided.

[Fig. 5] Fig. 5 is a schematic diagram illustrating the positional relationship between the tilted heat-emitting part shown in fig. 4 and a stick-type substrate.

[Fig. 6] Fig. 6 is a schematic diagram showing a model for deriving the tilt of the heat-emitting part when the hermetic member is provided.

[DESCRIPTION OF EMBODIMENTS]

Preferred embodiments of the present invention will be described in detail below with reference to the appended drawings. It should be noted that in the specification and the drawings, duplicate descriptions are omitted by using the same reference signs to denote constituent elements having substantially the same functional configuration.

<1. Example configuration of inhalation device>

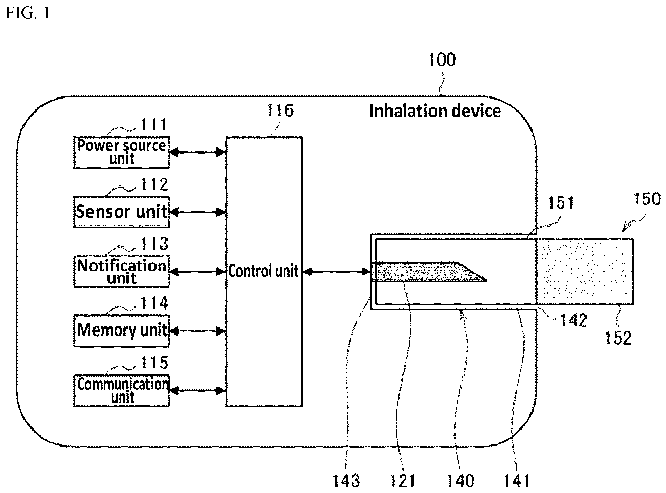

A configuration example of an inhalation device according to an embodiment of the present invention will be described first of all. The inhalation device according to this configuration example is capable of generating an aerosol by heating a substrate that contains an aerosol source from inside the substrate. This configuration example will be described below with reference to fig. 1.

Fig. 1 is a schematic diagram illustrating schematically a configuration example of an inhalation device. As shown in fig. 1, an inhalation device 100 according to this configuration example comprises a power source unit 111, a sensor unit 112, a notification unit 113, a memory unit 114, a communication unit 115, a control unit 116, a heat-emitting part 121, and a holding part 140. A user inhales with the inhalation device 100 in a state in which a stick-type substrate 150 is accommodated in the holding part 140. The components will be described in order below.

It should be noted that the inhalation device 100 and the stick-type substrate 150 cooperate to generate an aerosol to be inhaled by the user. As such, the combination of the inhalation device 100 and the stick-type substrate 150 may be considered to be an aerosol generation system.

The power source unit 111 stores electric power. The power source unit 111 then supplies electrical power to each component of the inhalation device 100. The power source unit 111 may be configured, for example, by a rechargeable battery such as a lithium ion secondary battery. The power source unit 111 may be charged by being connected to an external power source by means of a USB (universal serial bus) cable or the like. Furthermore, the power source unit 111 may also be charged by means of wireless power transmission technology, without being connected to a power transmission-side device. Additionally, the power source unit 111 may be provided so as to be removable from the inhalation device 100, and may be provided so as to be replaceable with a new power source unit 111.

The sensor unit 112 detects various types of information relating to the inhalation device 100 and outputs the detected information to the control unit 116. As an example, the sensor unit 112 is configured by a pressure sensor such as a capacitor microphone, a flow rate sensor or a temperature sensor. In this way, when the sensor unit 112 has detected a numerical value associated with inhalation by a user, the sensor unit 120 may then output, to the control unit 116, information indicating that the user has inhaled. As another example, the sensor unit 112 may be configured by an input device, such as a button or switch, for receiving input of information from the user. The sensor unit 112 may especially comprise a button for instructing starting/stopping of aerosol generation. In this way, the sensor unit 112 may output to the control unit 116 the information input by the user. As a further example, the sensor unit 112 may be configured by a temperature sensor for detecting the temperature of the heat-emitting part 121. In this way, the sensor unit 112 may determine the temperature of the stick-type substrate 150 accommodated in the holding part 140 by detecting the temperature of the heat-emitting part 121 on the basis of an electrical resistance value of the heat-emitting part 121, for example.

The notification unit 113 notifies the user of the information. As an example, the notification unit 113 is configured by a light-emitting device such as an LED (light-emitting diode). In this way, when the power source unit 111 is in a state of requiring charging, when the power source unit 111 is in the process of charging, or when an abnormality has occurred in the inhalation device 100, etc., the notification unit 113 may emit light in a different light emission pattern for each case. The light emission patterns as referred to here generally include colors and timing of illumination/extinguishing. Instead of or as well as the light-emitting device, the notification unit 113 may also be configured by a display device for displaying images, a sound output device for outputting sounds, or a vibration device which vibrates, etc. Additionally, the notification unit 113 may notify information indicating that inhalation by the user is possible. The information indicating that inhalation by the user is possible may be notified when the temperature of the stick-type substrate 150 heated by means of the heat-emitting part 121 has reached a predetermined temperature.

The memory unit 114 stores various types of information for the operation of the inhalation device 100. The memory unit 114 is configured by a non-volatile storage medium such as a flash memory, for example. Information relating to the operating system (OS) of the inhalation device 100, such as information relating to control performed by the control unit 116 of various types of components, is an example of the information stored in the memory unit 114. Another example of the information stored in the memory unit 114 is information relating to inhalation by the user, such as number of inhalations, times of inhalation, or cumulative inhalation time.

The communication unit 115 is a communication interface for sending and receiving information between the inhalation device 100 and another device. The communication unit 115 performs communication conforming to any wired or wireless communication standard. Examples of communication standards which may be used include wireless LAN (local area network), wired LAN, Wi-Fi (registered trademark), and Bluetooth (registered trademark), etc. As one example, the communication unit 115 may send information relating to inhalation by the user to a smartphone in order to cause the smartphone to display the information relating to inhalation by the user. As another example, the communication unit 115 may receive new OS information from a server in order to update the OS information stored in the memory unit 114.

The control unit 116 functions as an arithmetic processing device and a control device, and controls overall operation within the inhalation device 100 in accordance with various programs. The control unit 116 is realized by a CPU (central processing unit) or an electronic circuit such as a microprocessor, for example. The control unit 116 may also include a ROM (read-only memory) for storing programs and computation parameters, etc. which are used, and a RAM (random access memory) for temporarily storing suitably changing parameters, etc. The inhalation device 100 implements various types of processing on the basis of control performed by the control unit 116. Examples of processing controlled by the control unit 116 include: supply of electricity from the power source unit 111 to other components; charging of the power source unit 111; detection of information by the sensor unit 112; notification of information by the notification unit 113; storage and reading of information by the memory unit 114; and sending/receiving of information by the communication unit 115. Other processing implemented by the inhalation device 100, such as processing based on input of information to each component and information output from each component, are also controlled by means of the control unit 116.

The holding part 140 has an internal space 141, and holds the stick-type substrate 150 while accommodating a portion of the stick-type substrate 150 in the internal space 141. The holding part 140 has an opening 142 allowing the internal space 141 to communicate with the outside, and holds the stick-type substrate 150 which has been inserted into the internal space 141 from the opening 142. For example, the holding part 140 is a cylindrical body comprising the opening 142 and a bottom portion 143 serving as a bottom surface, and defines a columnar internal space 141. The holding part 140 is configured so that the inner diameter of at least part of the cylindrical body in a height direction is smaller than the outer diameter of the stick-type substrate 150, and is capable of holding the stick-type substrate 150, which has been inserted into the internal space 141, so as to press the stick-type substrate 150 from the outer circumference thereof. The holding part 140 also has a function for defining a flow path for air passing through the stick-type substrate 150. An air inflow hole which is an inlet for air into the flow path is disposed in the bottom portion 143, for example. Meanwhile, the opening 142 forms an air outflow hole, which is an outlet for air from the flow path.

The stick-type substrate 150 is a substrate in the shape of a stick which generates an aerosol. The stick-type substrate 150 comprises a substrate portion 151 and a mouthpiece portion 152.

The substrate portion 151 includes an aerosol source. The aerosol source is atomized by heating so as to generate an aerosol. The aerosol source may be, for example, a tobacco-derived material such as shredded tobacco, or a processed product obtained by molding a tobacco raw material into a granular form, a sheet form, or a powder form. Furthermore, the aerosol source may also contain a non-tobacco-derived substance produced from a plant other than tobacco (e.g., mint or herb, etc.). When the inhalation device 100 is a medical inhaler, the aerosol source may contain a drug to be inhaled by a patient. It should be noted that the aerosol source is not limited to a solid, and may equally be a polyhydric alcohol such as glycerol or propylene glycol, or may be a liquid such as water, for example. In a state in which the stick-type substrate 150 is being held in the holding part 140, at least part of the substrate portion 151 is accommodated in the internal space 141 of the holding part 140.

The mouthpiece portion 152 is a member which is held in the user's mouth during inhalation. In a state in which the stick-type substrate 150 is being held in the holding part 140, at least part of the mouthpiece portion 152 protrudes from the opening 142. When the user then inhales with the mouthpiece portion 152, which protrudes from the opening 142, held in the mouth, air flows into the holding part 140 from the air inflow hole which is not depicted. The air which has flowed in passes through the internal space 141 of the holding part 140, that is, through the substrate portion 151, and reaches the user's mouth together with the aerosol generated from the substrate portion 151.

The heat-emitting part 121 heats the aerosol source to atomize the aerosol source, thereby generating the aerosol. The heat-emitting part 121 has a blade-like or pin-like form and is arranged so as to protrude into the internal space 141 from the bottom portion 143 of the holding part 140. When the stick-type substrate 150 is inserted into the holding part 140, the blade-like or pin-like heat-emitting part 121 therefore pierces the substrate portion 151 of the stick-type substrate 150 and is inserted inside the stick-type substrate 150. When the heat-emitting part 121 generates heat, the aerosol source contained in the stick-type substrate 150 is then heated from the inside of the stick-type substrate 150 and atomized, generating the aerosol. The heat-emitting part 121 generates heat when supplied with electricity from the power source unit 111. As an example, when the sensor unit 112 has detected that there has been predetermined user input, the heat-emitting part 121, which has been supplied with power, generates heat, and an aerosol is generated from the stick-type substrate 150 as a result of the temperature of the stick-type substrate 150 reaching a predetermined temperature. By this means, the inhalation device 100 is capable of enabling inhalation by the user. After this, the electrical supply to the heat-emitting part 121 may be stopped when the sensor unit 112 has detected that there has been predetermined user input. As another example, the aerosol may be generated by the heat-emitting part 121, which has been supplied with power, during a period in which the sensor unit 112 detects that there is inhalation by the user.

In the inhalation device 100 according to the present embodiment, proper arrangement of the hermetic member in the support structure of the heat-emitting part 121 protruding from the bottom portion 143 of the holding part 140 can prevent the ingress of moisture from the internal space 141 of the holding part 140 along the heat-emitting part 21. The support structure of such a heat-emitting part 121 will be described in detail below.

<2. Support structure of the heat-emitting part>

(2. 1. First configuration example)

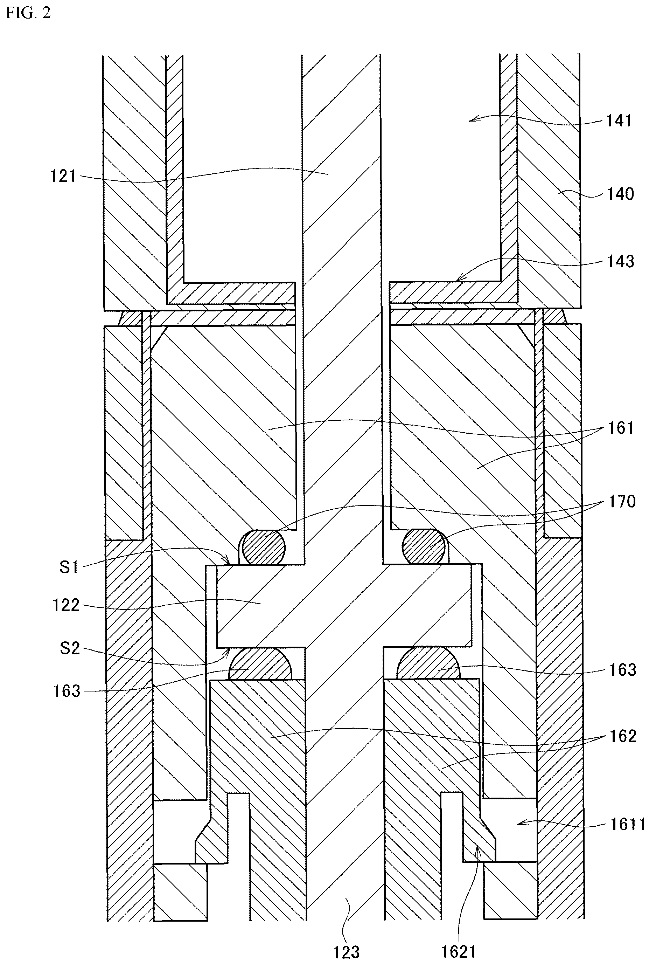

A first configuration example of the support structure of the heat-emitting part 121 will be described with reference to fig. 2. Fig. 2 is a schematic cross-sectional view showing the first configuration example of the support structure of the heat-emitting part 121.

As shown in fig. 2, the heat-emitting part 121 is supported by securing the flange part 122 which protrudes in a direction perpendicular to the extension direction of the heat-emitting part 121 with the first and second securing parts 161, 162.

Specifically, the heat-emitting part 121 constitutes a blade-like or pin-like heater for heating the stick-type substrate 150 inserted into the internal space 141 of the holding part 140. The heat-emitting part 121 protrudes into the internal space 141 through the bottom portion 143 from outside of the holding part 140, and is thereby inserted inside the stick-type substrate 150 held in the internal space 141. The heat-emitting part unit 121 may be, for example, a PTC (positive temperature coefficient) heater which generates heat by being energized.

The flange part 122 is a projection which projects in a direction orthogonal to an extension direction of the heat-emitting part 121, at one end of the heat-emitting part 121 on the opposite side to the other end protruding into the internal space 141 of the holding part 140. The flange part 122 is gripped in the extension direction of the heat-emitting part 121 by the first securing part 161 and the second securing part 162 via the hermetic member 170 and the horizontal support portion 163. The flange part 122 may be formed by a material that does not generate heat as a result of energization.

An extension 123 extends in the extension direction of the heat-emitting part 121 from a second face S2 on the opposite side to a first face S1 of the flange part 122 where the heat-emitting part 121 protrudes. The extension 123 is gripped by the second securing part 162 in a direction orthogonal to the extension direction of the extension 123. In this way, the heat-emitting part 121 can extend in a direction perpendicular to the bottom portion 143 of the holding part 140 as a result of the flange part 122 and the extension 123 being fixed by the first securing part 161 and the second securing part 162. Similarly to the flange part 122, the extension 123 may be formed by a material that does not generate heat as a result of energization.

The first securing part 161 and the second securing part 162 are provided opposite to the internal space 141 to the bottom portion 143 of the holding part 140 and support the heat-generating part 121 by gripping the flange part 122 and the extension 123. Specifically, the first securing part 161 supports the first face S1 of the flange part 122 on the side on which the holding part 140 is provided via the hermetic member 170. Meanwhile, the second securing part 162 supports, by way of the horizontal support portion 163, the second face S2 of the flange part 122 on the opposite side to the side on which the holding part 140 is provided. As a result, the flange part 122 is fixed by being gripped in the extension direction of the heat-emitting part 121 between the first securing part 161 and the second securing part 162.

Furthermore, the first securing part 161 is provided by a recessed structure which is open on one face on the opposite side to the side on which the holding part 140 is provided. The second securing part 162 is accommodated together with the flange part 122 inside the recessed structure of the first securing part 161, and has an overhang 1621 which becomes larger toward an inside face of the recessed structure of the first flange part 161. The second securing part 162 is mated by causing the overhang 1621 to protrude into a cutout 1611 provided in the side face of the first securing part 161, whereby the second securing part 162 is fixed so as not to become detached from the first securing part 161. It should be noted that the first securing part 161 may also serve as the housing of the inhalation device 100. According to this, the inhalation device 100 can be assembled more easily due to the reduction in component members.

The horizontal support portion 163 is provided between the second face S2 of the flange part 122 and the second securing part 162. The horizontal support portion 163 controls the tilt of the heat-emitting part 121 so that the heat-emitting part 121 protrudes perpendicularly into the internal space 141 of the holding part 140, by controlling the second face S2 of the flange part 122 so as to be parallel to the bottom portion 143 of the holding part 140. Specifically, the horizontal support portion 163 may be configured by a plurality of hemispherical portions or projecting structures, and may be provided so that peaks of the plurality of hemispheres or projecting structures are included on the same plane parallel to the bottom portion 143 of the holding part 140. In this way, the horizontal support portion 163 can ensure perpendicularity of the heat-emitting part 121 to the bottom portion 143 by supporting the second face S2 of the flange part 122 on a plane parallel to the bottom portion 143 of the holding part 140.

Here, the first securing part 161 and the holding part 140 are provided so as not to contact the side surface of the heat-emitting part 121. This is to prevent heat emitted from the heat-emitting part 121 conducting to the first securing part 161 and holding part 140 by contact, reducing the heating efficiency of the stick-type substrate 150. However, in such a case, there will be a gap between the heat-emitting part 121 and the first securing part 161/holding part 140. Since the gap created on the side of the heat-emitting part 121 communicates with the space around the flange part 122 and the extension 123, if water is injected into the internal space 141 of the retention portion 140, moisture can enter the interior of the inhalation device 100 through the gap.

As shown in fig. 2, in the first configuration example, a ring-shaped hermetic member 170 is provided between the first face S1 of the flange part 122 and the first securing part 161. This allows the inhalation device 100 to seal the gap created in the side of the heat-emitting part 121 with the hermetic member 170. Accordingly, the inhalation device 100 is capable of preventing water that has poured into the internal space 141 from penetrating inside the inhalation device 100, and thus it is possible to improve the waterproofing properties.

The hermetic member 170 may be formed by an elastic material or metal material. For example, the hermetic member 170 may be an O-ring, a gasket, or a sealing washer, etc. formed from rubber, silicone resin, various types of organic resins, or aluminum, etc.

The hermetic member 170 is compressed and elastically deformed between the first face S1 of the flange part 122 and the first securing part 161. According to this, the hermetic member 170 can generate compressive stress between the flange part 122 and the first securing part 161, so that the hermetic member 170 can seal more tightly the space between the flange part 122 and the first securing part 161. Furthermore, the hermetic member 170 can further inhibit the tilt of the flange part 122 relative to the bottom portion 143 of the holding part 140, as the hermetic member can press the flange part 122 more strongly against the horizontal support 163 by compressive stress.

Furthermore, if the hermetic member 170 is gripped between the first face S1 of the flange part 122 and the first securing part 161, the inhalation device 100 can be assembled more easily.

For example, if the hermetic member 170 is provided between a side of the first securing part 161 and a side of another member such as a housing, there is the possibility that strong frictional forces may occur between the side of the first securing part 161 and the side of another member such as the housing during assembly of the support structure of the heat-emitting part 121 due to the hermetic member 170. In such a case, the difficulty of assembling the support structure of the heating element 121 may increase, or the first securing part 161 or the hermetic member 170 may be scratched, reducing the hermetic properties. On the other hand, when the hermetic member 170 is gripped between the first surface S1 of the flange part 122 and the first securing part 161, it is possible to assemble the support structure of the heat-emitting part 121 without creating friction. Thus, with the hermetic member 170 in the above arrangement, the inhalation device 100 can be assembled more easily and a loss of hermetic properties during assembly can be prevented.

The diameter of the ring-shaped hermetic member 170 may be 1-8 mm, for example, in view of the size of the heat-emitting part 121 and the inhalation device 100. If the diameter of the ring-shaped hermetic member 170 is in the above range, the hermetic member 170 is able to better seal the space between the flange part 122 and the first securing part 161.

The thickness of the hermetic member 170 may also be greater than or equal to the width between the flange part 122 and the first securing part 161. With the thickness of the hermetic member 170 greater than or equal to the width between the flange part 122 and the first securing part 161, the hermetic member 170 will be elastically deformed between the flange part 122 and the first securing part 161. Thus, the hermetic member 170 is able to generate compressive stress, so it is possible to seal more tightly the space between the flange part 122 and the first securing part 161. The thickness of the hermetic member 170 may be 1 mm or less, for example.

In the first configuration example, elastically deforming the hermetic member 170 between the flange part 122 and the first securing part 161 allows for a stronger seal between the flange part 122 and the first securing part 161 using the restoring force of the hermetic member 170. Thus, the hermetic member 170 can improve the waterproofing properties of the inhalation device 100 as the hermetic member can more reliably prevent moisture ingress from the internal space 141 of the holding part 140 into the interior of the inhalation device 100.

(2. 2. Second configuration example)

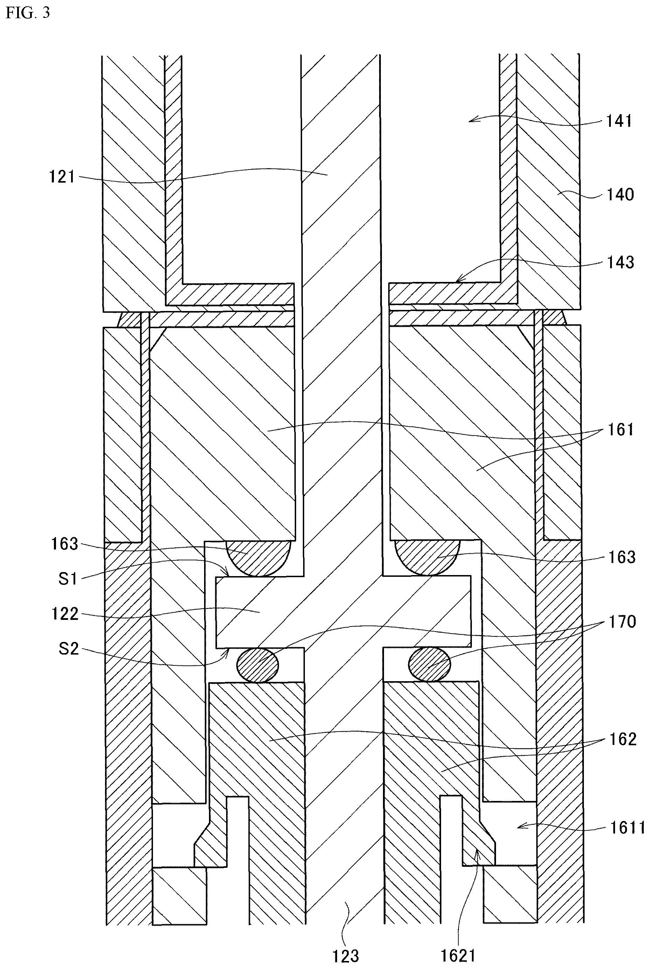

A second configuration example of the support structure of the heat-emitting part 121 will be described with reference to fig. 3. Fig. 3 is a schematic cross-sectional view showing a second configuration example of the support structure of the heat-emitting part 121.

As shown in fig. 3, the heat-emitting part 121 is supported by securing the flange part 122 which protrudes in a direction perpendicular to the extension direction of the heat-emitting part 121 with the first and second securing parts 161, 162. The heat-emitting part 121, the flange part 122, the extension 123, the first securing part 161, and the second securing part 162 are substantially the same as in the first configuration example and will therefore not be described again.

In the second configuration example, a ring-shaped hermetic member 170 is provided between the second face S2 of the flange part 122 and the second securing part 162. Meanwhile, the horizontal support portion 163 is provided between the first face S1 of the flange part 122 and the first securing part 161. The horizontal support portion 163 controls the tilt of the heat-emitting part 121 so that the heat-emitting part 121 protrudes perpendicularly into the internal space 141 of the holding part 140, by controlling the first face S1 of the flange part 122 so as to be parallel to the bottom portion 143 of the holding part 140. That is, in the second configuration example, the positions of the horizontal support 163 and the hermetic member 170 are swapped relative to the first example of configuration.

The hermetic member 170 is a ring-shaped member formed by an elastic material or metal material. For example, the hermetic member 170 may be an O-ring, a gasket, or a sealing washer, etc. formed from rubber, silicone resin, various types of organic resins, or aluminum, etc.

The hermetic member 170 is compressed and elastically deformed between the second face S2 of the flange part 122 and the second securing part 162. According to this, the hermetic member 170 can generate compressive stress between the flange part 122 and the second securing part 162, so that the hermetic member 170 can seal more tightly the space between the flange part 122 and the second securing part 162. Accordingly, the hermetic member 170 is capable of preventing water that has poured into the internal space 141 from penetrating inside the inhalation device 100, and thus it is possible to improve the waterproofing properties of the inhalation device 100. Furthermore, the hermetic member 170 can further inhibit the tilt of the flange part 122 relative to the bottom portion 143 of the holding part 140, as the hermetic member can press the flange part 122 more strongly against the horizontal support 163 by compressive stress.

<3. Action And Effects>

As noted above, in the inhalation device 100 according to this embodiment, a hermetic member 170 is provided between the flange part 122 of the heat-emitting part 121 and the first securing part 161/second securing part 162 gripping the flange part 122. The inhalation device 100 according to the present embodiment is able to provide a hermetic member 170 at any position between the flange part 122 and the first securing part 161/second securing part 162, to prevent moisture injected into the inner space 141 from entering the interior of the inhalation device 100.

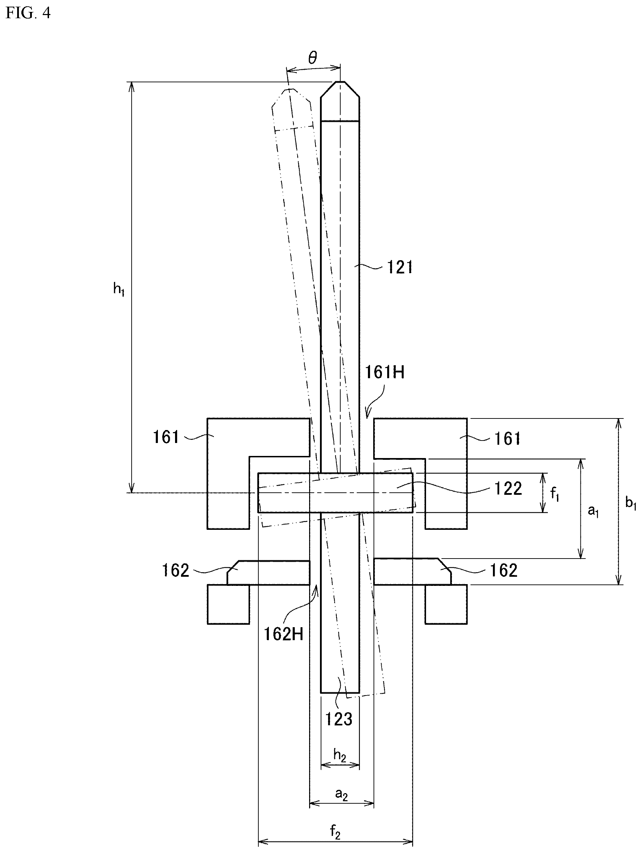

The inhalation device 100 according to this embodiment is also able to control the tilt of the heat-emitting part 121 with greater precision. According to this, the inhalation device 100 can mitigate the machining precision required for each part of the support structure of the heating-emitting part 121. The actions and effects will be described with reference to fig. 4-6.

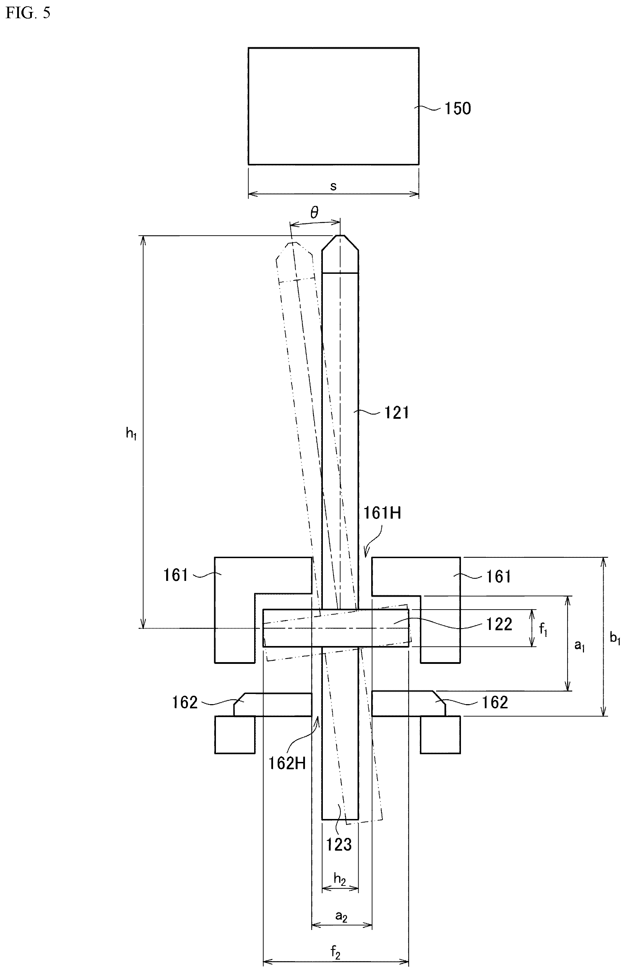

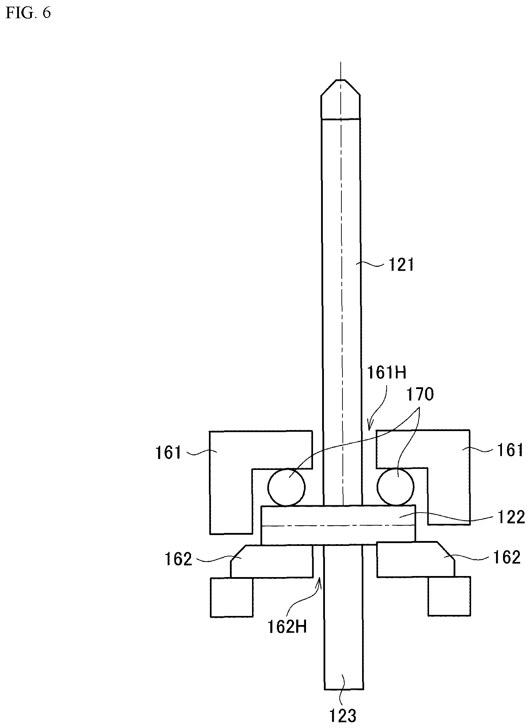

Fig. 4 is a schematic diagram showing a model for deriving the tilt of the heat-emitting part 121 when the hermetic member 170 is not provided. Fig. 5 is a schematic diagram illustrating the positional relationship between the tilted heat-emitting part 121 shown in fig. 4 and the stick-type substrate 150. Fig. 6 is a schematic diagram showing a model for deriving the tilt of the heat-emitting part 121 when the hermetic member 170 is provided. In fig. 4-6, each part is omitted relative to fig. 2, and the holding part 140 side is referred to as the upper side and the opposite side to the holding part 140 is referred to as the lower side.

When the dimensions of each of the heat-emitting part 121, the first securing part 161, and the second securing part 162 are set, as shown in fig. 4, the tilt angle θ of the heat-emitting part 121 can be expressed in the following formula (1) or formula (2). The following formula (1) represents the tilt angle θ of the heat-emitting part 121 when the flange part 122, the first securing part 161, and the second securing part 162 interfere with each other. The formula (2) represents the tilt angle θ of the heating-emitting part 121 when the heating-emitting part 121 and the extension 123, the first securing part 161, and the second securing part 162 interfere with each other.

Note that $a_1$ is the distance from the lower end of a first aperture 161H of the first securing part 161 through which the heat-emitting part 121 passes to the upper end of the second aperture 162H of the second securing part 162 through which the extension 123 passes. $b_1$ is the distance from the upper end of the first aperture 161H to the lower end of the second aperture 162H. $a_2$ is the width of the second aperture 162H and $h_2$ is the width of the heat-emitting part 121 and the extension 123. $f_1$ is the thickness of the flange part 122 in the extension direction of the heat-emitting part 121 and $f_2$ is the width of the flange part 122 in a direction perpendicular to the extension direction of the heat-emitting part 121.

[Math. 1]

$$\theta = \sin^{-1}\left(\frac{a_1}{\sqrt{f_2^2+f_1^2}}\right) - \tan^{-1}\left(\frac{f_1}{f_2}\right) \ \ ...\,(1)$$

$$\theta = \tan^{-1}\left(\frac{a_2}{b_1}\right) - \tan^{-1}\left(\frac{h_2}{\sqrt{a_2^2+b_1^2}}\right) \ \ ...\,(2)$$

That is, there is a possibility that the heat-emitting part 121 may rattle between each of the heat-emitting part 121, the first securing part 161, and the second securing part 162 and, depending on the machining accuracy, the heat-emitting part may tilt from the vertical direction by the angle θ expressed in the above formula (1) or (2). If the heat-emitting part 121 extends tilted from the vertical direction of the bottom portion 143 of the holding part 140 in this way, the heat-emitting part 121 may protrude out of the inserted stick-type substrate 150.

Here, as shown in fig. 5, if defining the width of the stick-type substrate 150 as s, the tilt angle $\theta_s$ at which the heat-emitting part 121 protrudes out of the stick-type substrate 150 is expressed by the following formula (3). [Math. 2]

$$\theta_s = sin^{-1}(\frac{1}{h_1} * \frac{s-h_2}{2}) \quad ... (3)$$

If s=7 mm, $h_1$=30 mm, $h_2$=1 mm, then $\theta_s$ is 5.73°. That is, when the tilt angle θ of the heat-emitting part 121 exceeds $\theta_s$= 5.73°, the heat-emitting part 121 will protrude out of the stick-type substrate 150.

When the dimensional tolerance is 0.2 mm, if defining the clearance between the heat-emitting part 121 and the first securing part 161 as 0.1 mm, then $a_2$=1.3 mm. If defining the clearance between the flange part 122, the first securing part 161, and the second securing part 162 as 0.1 mm, assuming a thickness of 1 mm for each of the first securing part 161 and the second securing part 162, $b_1$=3.3 mm. In such a case, if $f_1$=1 mm, then in the above equation (2), θ=5.75°, and thus the heat-emitting part 121 protrudes out from the stick-type substrate 150.

When the dimensional tolerance is 0.1 mm, if defining the clearance between the heat-emitting part 121 and the first securing part 161 as 0.1 mm, then $a_2$=1.2 mm. If defining the clearance between the flange part 122, the first securing part 161, and the second securing part 162 as 0.1 mm, assuming a thickness of 1 mm for each of the first securing part 161 and the second securing part 162, $b_1$=3.2 mm. In such a case, if $f_1$=1 mm, then in the above equation (2), θ=4.25°, and thus the heat-emitting part 121 does not protrude out from the stick-type substrate 150.

As shown in figs. 4 and 5, when the hermetic member 170 is not provided, the heat-emitting part 121 will have a different tilt angle depending on the rattling between the parts and the size of the dimensional tolerance. Therefore, in order to reliably insert the heat-emitting part 121 into the stick-type substrate 150 and heat the stick-type substrate 150 efficiently, it is important to increase the processing and assembly precision.

As shown in fig. 6, in the inhalation device 100 according to the present embodiment, the hermetic member 170 is provided to make it possible to absorb rattling between parts caused the elastic deformation of the hermetic member 170, and the size of the dimensional tolerances. Thus, the inhalation device 100 can absorb variations in machining accuracy and assembly accuracy, thus making it possible to reduce manufacturing costs.

Furthermore, in the inhalation device 100 according to this embodiment, the flange part 122 can be pressed against the horizontal support portion 163 by the compressive stress generated in the elastically deformed hermetic member 170. Thus, the inhalation device 100 can more easily fix the flange part 122 in parallel to the bottom portion 143 of the holding part 140. Thus, the inhalation device 100 allows the stick-type substrate 150 to be heated more efficiently due to the heat-emitting part 121 being able to be inserted more reliably without protruding from the stick-type substrate 150.

Preferred embodiments of the present invention were described in detail above with reference to the appended drawings, but the present invention is not limited to those examples. It is obvious that a person having ordinary knowledge in the technical field to which the present invention belongs will be able to conceive of a number of variant examples or modified examples within the scope of the technical concept disclosed in the claims, and any such variant examples or modified examples are naturally understood to fall within the technical scope of the present invention.

It should be noted that the following configurations also fall within the technical scope of the present invention.

(1) An aerosol generation device comprising: a holding part having a cylindrical structure, an aerosol-generating substrate being inserted into the interior of the cylindrical structure;

a heat-emitting part that protrudes into the cylindrical structure through a bottom surface of the cylindrical structure from the exterior thereof, the heat-emitting part heating the aerosol-generating substrate;
a flange part that juts out from the heat-emitting part at the exterior of the cylindrical structure in a direction orthogonal to an extension direction of the cylindrical structure;
a securing part that grips the flange part in the extension direction of the heat-emitting part; and
a hermetic member that is provided between the flange part and the securing part, the hermetic member hermetically sealing a gap communicating from the interior of the cylindrical structure along the heat-emitting part.

(2) The aerosol generation device as disclosed in (1), wherein the hermetic member generates compressive stress in the extension direction of the heat-emitting part.

(3) The aerosol generation device as disclosed in (2), wherein the hermetic member is made of an elastic material or a metallic material.

(4) The aerosol generation device as disclosed in any one of (1) to (3), wherein the hermetic member is ring-shaped.

(5) The aerosol generation device as disclosed in (4), wherein the diameter of the hermetic member is 1-8 mm.

(6) The aerosol generation device as disclosed in any one of (1) to (5), wherein a width between the flange part of the gap and the securing part is less than or equal to a thickness of the hermetic member.

(7) The aerosol generation device as disclosed in (6), wherein the thickness of the hermetic member is 1 mm or less.

(8) The aerosol generation device as disclosed in any one of (1) to (7), wherein the securing part includes a first securing part spaced apart from the heat-emitting part and provided on the same side as the holding part with respect to the flange part, and a second securing part provided on the opposite side of the holding part with respect to the flange part and gripping the heat-emitting part in a direction orthogonal to the extension direction of the heat-emitting part.

(9) The aerosol generation device as disclosed in (8), wherein the flange part is gripped between the first securing part and the second securing part.

[REFERENCE SIGNS LIST]

100 inhalation device
121 heat-emitting part
122 flange part
123 extension
140 holding part
141 internal space
142 opening
143 bottom portion
150 stick-type substrate
151 substrate portion
152 mouthpiece portion
161 first securing part
162 second securing part
163 horizontal support portion
170 hermetic member

**Claims**

1. An aerosol generation device comprising: a holding part having a cylindrical structure, an aerosol-generating substrate being inserted into the interior of the cylindrical structure;

   a heat-emitting part that protrudes into the cylindrical structure through a bottom surface of the cylindrical structure from the exterior thereof, the heat-emitting part heating the aerosol-generating substrate;
   a flange part that juts out from the heat-emitting part at the exterior of the cylindrical structure in a direction orthogonal to an extension direction of the cylindrical structure;
   a securing part that grips the flange part in the extension direction of the heat-emitting part; and
   a hermetic member that is provided between the flange part and the securing part, the hermetic member hermetically sealing a gap communicating from the interior of the cylindrical structure along the heat-emitting part.

2. The aerosol generation device as claimed in claim 1, wherein the hermetic member generates compressive stress in the extension direction of the heat-emitting part.

3. The aerosol generation device as claimed in claim 2, wherein the hermetic member is made of an elastic material or a metallic material.

4. The aerosol generation device as claimed in any one of claims 1 to 3, wherein the hermetic member is ring-shaped.

5. The aerosol generation device as claimed in claim 4, wherein the diameter of the hermetic member is 1-8 mm.

6. The aerosol generation device as claimed in any one of claims 1 to 5, wherein a width between the flange part of the gap and the securing part is less than or equal to a thickness of the hermetic member.

7. The aerosol generation device as claimed in claim 6, wherein the thickness of the hermetic member is 1 mm or less.

8. The aerosol generation device as claimed in any one of claims 1 to 7, wherein the securing part includes a first securing part spaced apart from the heat-emitting part and provided on the same side as the holding part with respect to the flange part, and a second securing part provided on the opposite side of the holding part with respect to the flange part and gripping the heat-emitting part in a direction orthogonal to the extension direction of the heat-emitting part.

9. The aerosol generation device as claimed in claim 8, wherein the flange part is gripped between the first securing part and the second securing part.

FIG. 1

FIG. 2

FIG. 3

FIG. 4

FIG. 5

FIG. 6

## INTERNATIONAL SEARCH REPORT

| International application No. |
| --- |
| **PCT/JP2022/040964** |

| A. | CLASSIFICATION OF SUBJECT MATTER |
| --- | --- |

*A24F 40/46*(2020.01)i
FI:   A24F40/46

According to International Patent Classification (IPC) or to both national classification and IPC

| B. | FIELDS SEARCHED |
| --- | --- |

Minimum documentation searched (classification system followed by classification symbols)

A24F40/46

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | JP 2020-528737 A (PHILIP MORRIS PRODUCTS S. A.) 01 October 2020 (2020-10-01)<br>entire text, all drawings | 1-9 |
| A | JP 2020-527040 A (KT&G CORP.) 03 September 2020 (2020-09-03)<br>entire text, all drawings | 1-9 |
| A | CN 212574138 U (HUBEI CHINA TOBACCO INDUSTRY CO., LTD.) 23 February 2021 (2021-02-23)<br>entire text, all drawings | 1-9 |
| A | CN 210642444 U (HUIZHOU PEIGESI TECHNOLOGY CO., LTD.) 02 June 2020 (2020-06-02)<br>entire text, all drawings | 1-9 |
| A | CN 213908500 U (CHINA TOBACCO HUNAN INDUSTRIAL CO., LTD.) 10 August 2021 (2021-08-10)<br>entire text, all drawings | 1-9 |
| A | US 2016/0073692 A1 (FONTEM HOLDINGS 2 B. V.) 17 March 2016 (2016-03-17)<br>entire text, all drawings | 1-9 |

☐ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **06 December 2022** | **20 December 2022** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT
### Information on patent family members

International application No.

**PCT/JP2022/040964**

| Patent document cited in search report | | | Publication date (day/month/year) | Patent family member(s) | | | Publication date (day/month/year) |
|---|---|---|---|---|---|---|---|
| JP | 2020-528737 | A | 01 October 2020 | US | 2020/0178605 | A1 | |
| | | | | entire document | | | |
| | | | | WO | 2019/020826 | A1 | |
| | | | | EP | 3657967 | A1 | |
| | | | | CN | 110913711 | A | |
| | | | | KR | 10-2020-0036812 | A | |
| JP | 2020-527040 | A | 03 September 2020 | US | 2020/0086068 | A1 | |
| | | | | entire document | | | |
| | | | | WO | 2019/050131 | A1 | |
| | | | | EP | 3556230 | A2 | |
| | | | | KR | 10-2018-0070453 | A | |
| | | | | CN | 207836767 | U | |
| CN | 212574138 | U | 23 February 2021 | (Family: none) | | | |
| CN | 210642444 | U | 02 June 2020 | (Family: none) | | | |
| CN | 213908500 | U | 10 August 2021 | (Family: none) | | | |
| US | 2016/0073692 | A1 | 17 March 2016 | WO | 2016/042409 | A1 | |
| | | | | EP | 3193643 | A1 | |
| | | | | CN | 106998812 | A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020528737 A